(19) Europäisches Patentamt
European Patent Office
Office européen des brevets

(11) Publication number: **0 000 078**
**B1**

(12) # EUROPEAN PATENT SPECIFICATION

(21) Application number: 78200017.8

(22) Date of filing: 01.06.78

(51) Int. Cl.³: C 07 F 9/65, C 07 D 499/68

(54) **Preparation of salts of hydroxyphosphinylureidobenzylpenicillins.**

(30) Priority: 01.06.77 GB 23227/77

(43) Date of publication of application: 20.12.78 Bulletin 78/01

(45) Publication of the grant of the European patent: 23.07.80 Bulletin 80/15

(84) Designated Contracting States: BE CH DE FR GB LU NL SE

(56) References cited: None

(73) Proprietor: Gist-Brocades N.V.
Wateringseweg 1 P.O. Box 1
NL - 2600 MA Delft (NL)

(72) Inventor: Akkerboom, Piet Johannes
Dr. J. W. Palthelaan 48
NL - 2712 RT Zoetermeer (NL)
Löwer, Geertruida Johanna
Rijksweg Noord 1D2
NL - 6162 AA Geleen (NL)
Timp, Willem Jacob
W. H. van Leeuwenlaan 84
NL - 2613 ZG Delft (NL)

(74) Representative: Broekhuijsen, Robbert
c/o Gist-Brocades N.V. Wateringseweg 1 P.O. Box 1
NL - 2600 MA Delft (NL)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 000 078 B1

Courier Press, Leamington Spa, England.

# Preparation of salts of hydroxyphosphinylureidobenzylpenicillins

This invention relates to a new process for the preparation of salts of hydroxyphosphinylureidobenzylpenicillins.

Processes for the preparation of hydroxyphosphinylureidobenzylpenicillins are already known. For example, British Patent Specification No. 1,464,551 describes, in Example 26, the reaction of silylated D - 6 - ($\alpha$ - amino) - benzylcarboxamidopenicillanic acid (ampicillin) with chloro(ethoxy)phosphinylisocyanate[$C_2H_5O$—P(O)(Cl)NCO] in an organic solvent at a temperature of −65 to −70°C. The reaction mixture obtained is poured into iced water, simultaneously being neutralised with aqueous sodium hydroxide. After extracting the aqueous phase of the two-phase system formed with an organic solvent, the extract is evaporated, yielding about 5.5% of D - 6 - [$\alpha$-{3 - hydroxy(ethoxy) - phosphinyl) - ureido} benzylcarboxamido]penicillanic acid. This compound may be converted into a salt which appears to be more stable than the acid, but nevertheless, the yield remains low, if calculated on the ampicillin compound used as starting material. Using the same method but using as starting material D - 7 - ($\alpha$ - amino) - benzylcarboxamido-desacetoxycephalosporanic acid (cephalexin) a much higher yield is obtained, being 61%, according to Example 30 of the same Specification. The cephalosporin is apparently more stable than the corresponding penicillin.

The disodium salt of D - 6 - [$\alpha$ - {3 - (Hydroxy(ethoxy) - phosphinyl)ureido}benzylcarboxamido]penicillinic acid may also be prepared by another process as described in Example 35 of the above-mentioned Specification. Silylated ampicillin is reacted with benzyloxy(ethoxy)phosphinyl-isocyanate [$C_6H_5CH_2O(C_2H_5O)P$ - (O)NCO] and the compound formed, after separation, is reduced with hydrogen using palladium-on-charcoal as a catalyst, in the presence of sodium bicarbonate. Although this results in a much higher yield of the product, i.e. 50% based on the ampicillin starting material, the process has drawbacks in that palladium-on-charcoal is expensive and the reduction step takes a considerable length of time.

By using anhydrous D - 6 - ($\alpha$ - amino) - p - hydroxybenzyl - carboxamido-penicillanic acid (amoxicillin) as the starting material, as described in German "Offenlegungsschrift" No. 25 46 910, Example 5, it is possible to obtain the disodium salt of D - 6 - [$\alpha${3 - (hydroxy(ethoxy)phosphinyl)ureido} - p - hydroxy - benzylcarboxamido]penicillanic acid using a similar reduction method, with palladium-on-charcoal as a catalyst. A yield of 74% is obtained, calculated on the starting amoxicillin compound. However, the use of the expensive catalyst is a drawback of this process.

It has now been found that attractive yields of salts of hydroxyphosphinylureidobenzylpenicillins can be obtained (e.g. yields of 50% or more) without use of the expensive palladium-on-charcoal catalyst by a process which is similar to that disclosed in the above mentioned British Patent Specification No. 1,464,551, Example 26, but in which some modifications are adopted.

The present invention accordingly provides a process for the preparation of compounds of the general formula:

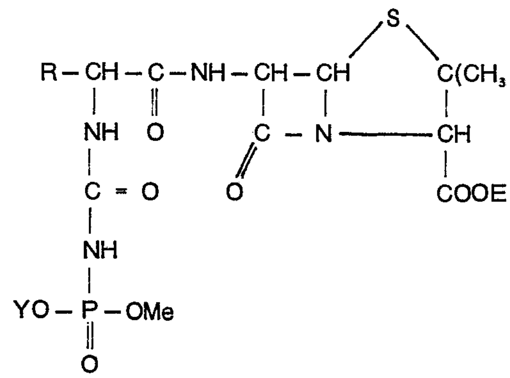

(I)

wherein R represents a phenyl group which may be substituted by one or two groups, which may be the same or different, selected from hydroxy, lower alkyl and lower alkoxy groups, Y represents a group Me or a lower alkyl, aryl(lower)alkyl or aryl group, in which the aryl group may be substituted by one or more lower alkyl groups, Me represents a metal cation, preferably an alkali metal cation (e.g. sodium or potassium) or alkaline earth metal cation (e.g. calcium), and E represents a hydrogen atom or a metal cation, which may be the same or different from the metal cation Me, which comprises reacting a compound of the general formula:—

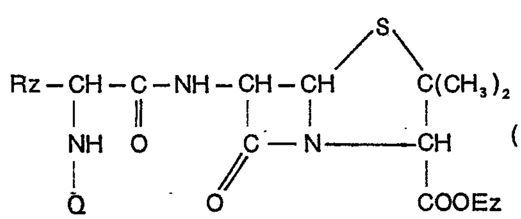

(II)

wherein Q represents a hydrogen atom or a silicon atom carrying substituents selected from lower alkyl, lower haloalkyl, aryl, aralkyl, or lower alkoxyalkyl groups and halogen atoms (preferably a trimethylsilyl group), Rz has the same significance as R as hereinbefore defined, with the proviso that any hydroxy group(s) present on the phenyl group is (or are) replaced by a group —Oz, wherein Qz has the same significance as Q as hereinbefore defined but excluding hydrogen, and Ez represents a

carboxyl-protecting group, e.g. a group Qz as hereinbefore defined, with a compound having the general formula:

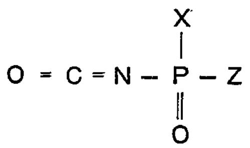

(III)

wherein Z represents a halogen, preferably a chlorine atom, and X represents a group OY, wherein Y is as hereinbefore defined, or a group Z as hereinbefore defined, at a temperature below 0°C, preferably from about −90° to about −40°C, more preferably from about −80° to about −60°C, under anhydrous conditions in an organic solvent medium, e.g. methylene chloride or ethyl acetate, characterised by carefully hydrolysing the product thus obtained either with an amount of water just sufficient to remove any protecting groups present in the intermediate product and to hydrolyse the group(s) Z to hydroxy group(s) and, still under anhydrous or substantially anhydrous conditions, converting hydroxy group(s) formed by hydrolysis of group(s) Z into group(s) OMe, wherein Me is as hereinbefore defined, by means of an organic acid salt having a salt-forming cation Me, preferably an alkanoic salt, the alkanoyl group of which contains 1 to 20 carbon atoms, preferably 5 to 10 carbon atoms or, in which case the organic solvent used is one insoluble or substantially insoluble in water, with up to 100% excess, preferably 20 to 60% excess, of water, washing the mixture with water at a pH-value of 0 to 3, preferably 1 to 2, extracting the organic layer formed with water at a pH-value of 5 to 8, preferably 6.5 to 7.5 by means of adding a hydroxide or a salt having a salt-forming cation Me, thus converting hydroxy group(s) formed by hydrolysis of groups Z into groups OMe and Ez into Me, wherein Me is as hereinbefore defined.

The term "lower" as used in connection with alkyl and alkoxy groups means that the groups in question contain 1 to 6 carbon atoms.

The salt formation, when carried out in an organic medium is preferably carried out in organic solvents having moderately polar properties, such as ethanol, butanol, acetone, ethyl acetate, methyl isobutyl ketone, methylene chloride or mixtures of two or more of these solvents. Examples of useful salts are salts of acetic acid, propionic acid, butyric acids, pentanoic acids, hexanoic acids, heptanoic acids, octanoic acids, stearic acids, etc. Preferably salts of 2-ethylhexanoic acid are used.

Due to the difference in $pK_a$ of the P—OH and COOH groups salt formation on addition of an organic acid salt will at first take place exclusively at the POH group(s), whereupon the formed partial salt precipitates and because of this the carboxylate ion will not be formed. Therefore large amounts of the organic acid salt excessive to the amount calculated for salt formation of the P—OH group(s) are useless and should normally be avoided in order not to contaminate the final product unnecessarily. The precipitated partial salt is collected and converted into a di- or tri-salt, as the case may be, in any conventional manner, e.g. by means of aqueous sodium carbonate.

In the case the hydrolysed compound is washed with water before salt formation, care must be taken that the compound has been dissolved in an organic medium not or substantially not soluble in water, in order that the compound remains in the organic phase during this washing. Examples of suitable solvents are methylene chloride, ethyl acetate, etc., but solvents like acetone should then be avoided. The water may be acidified by a solution of an inorganic acid, such as hydrochloric, phosphoric, nitrile and sulphuric acid, etc. Hydrochloric acid is conveniently used. The salt formation afterwards may be effected by the use of solutions of a hydroxide or a salt of cations hereinbefore defined, such as sodium hydroxide.

The starting compounds of formula II may be prepared by methods known per se. The phosphinylisocyanates of formula III may be prepared, for example, as described by Narbut et al, Zh. Obshch. Khim. 38 (1968) page 1321 and Gubnitskaya et al, Zh. Obshch.Khim. (1970) page 1205.

By the term "methods known per se" as used in this specification is meant methods heretofore used or described in the chemical literature.

The cations E and Me are preferably nontoxic, pharmaceutically acceptable cations for penicillins, preferably sodium, potassium or calcium.

The starting penicillin compounds from which the compounds of general formula II are prepared contain a carboxylic acid or a carboxylic salt group and may contain other groups which may also interfere with the reaction. These groups are protected by a group Qz as hereinbefore defined by methods known per se. Preferably silyl esters are prepared by reacting the free carboxyl group with, e.g. trimethylchlorosilane, N,O-bistrimethylsilylacetamide, trimethylsilylacetamide, dimethyldichlorosilane, bistrimethylsilylurea, bistrimethylsilylcarbamate or bistrimethylsilylsulphamate. When silylhalo compounds are used it is preferred to carry out the silylation reaction in the presence of an acid-binding compound such as triethylamine or ethylenediamine.

An advantage of the process of the present invention over methods previously disclosed in specification as heretobefore mentioned for the preparation of hydroxyphosphinylureidobenzylpenicillins is that the reactions involved can all be carried out in one reaction vessel.

The compounds of general formula I prepared by the process of the invention, show

antibiotic activity, as described in the aforementioned British Patent Specification No. 1,464,551 and German "Offenlegungsschrift" No. 25 46 910.

The invention is illustrated by the following Examples.

Example 1.

Preparation of the disodium salt and the sodium potassium double salt of D - 6{$\alpha$ - [3 - (hydroxy(ethoxy)phosphinyl) - ureido] - *p* - hydroxybenzylcarboxamido}penicillanic acid.

D - 6 - ($\alpha$ - amino) - *p* - hydroxybenzylcarboxamido penicillanic acid (amoxicillin) (36.9 g, 0.10 mole) is silylated with 53.4 ml (0.22 mole) of N,O-bistrimethylsilylacetamide in 250 ml of methylene chloride as solvent. After cooling the solution to about −70°C a solution of 17 g (0.10 mole) of $(C_2H_5O)PO(Cl)NCO$ (ethylchlorophosphorusisocyanatidate) in 250 ml of dry acetone is added dropwise. After completion of the addition the mixture is stirred for 1½ hours and a mixture of 5.8 ml (0.32 mole) of water, 8.1 ml (0.10 mole) of pyridine and 250 ml of dry acetone are added at a temperature of −70°C. The temperature of the reaction mixture is then allowed to rise quickly to −40°C and then slowly to −10°C, during a period of 2 hours, in order to complete the hydrolysis of the chloride and protective silyl groups. 2 g of activated carbon are added and after stirring for 10 minutes the mixture is filtered. A solution of 16.8 g (0.10 mole) of sodium 2-ethylhexanoate in 200 ml of dry acetone is added to the filtrate at +5°C, and the mixture is stirred for 10 minutes at the same temperature, and for a further 15 minutes at ambient temperature. A precipitate is formed which is filtered off and suspended in 1½ litre of dry acetone. The mixture is stirred mechanically for 45 minutes and the precipitate is then filtered off. This operation is repeated, resulting in a much purer product. The precipitate from the repeated operation is suspended in 750 ml of water. The suspension is cooled by the exterior application of ice and nitrogen bubbled through the mixture, and the precipitate dissolved by the addition of 5.3 g of sodium bicarbonate at pH 6.8. By freeze-drying the solution 33 g (58%) of the title disodium salt are obtained, containing only a trace of sodium 2-ethylhexanoate. The physicochemical properties of the compound obtained are as follows:—

IR (KBr-disc, values in $cm^{-1}$): about 3200—3600 (broad and intensive), 2975, 2930 (sh), 1765, ± 1650—1670, ± 1610, 1550 (sh), 1515, 1460, 1400 1375 (sh), ± 1325, about 1240(broad), 1180, 1135, 1085, 1050, 955, 900, 770.

PMR (about 5:1 mixture of $d_6$-DMSO and $DCO_2D$, DSS as reference, $\delta$-values in ppm): 1.25 (centre of two close triplets, J ≈ 7.5 cps), 1.46 (s) and 1.59 (s) all together 9H; about 3.75 to 4.1 (multiplet, 2H), 4.22 (s, 1H), 5.42 (s) and about 5.3 to 5.6 (broadened AB-q) together 3H; 6.65 to 7.35 (q-like, J ≈ 8.5 cps, 4H).

The physicochemical properties are identical to those of the compound prepared in Example 5 of German "Offenlegungsschrift" No. 25 46 910.

The sodium potassium double salt is prepared in the same manner, but instead of sodium 2-ethylhexanoate, 10 g (0.10 mole) of potassium acetate, dissolved in butanol, are added. A precipitate is formed slowly. The precipitate is treated with sodium bicarbonate in the manner described above, yielding 26 g (45%) of the title sodium potassium compound having physicochemical properties identical to those of the disodium salt given above.

The disodium salt may also be prepared by using a sodium stearate suspension in acetone, instead of the sodium 2-ethylhexanoate. A white powder is obtained consisting of the monosodium salt containing a fair amount of sodium stearate. 3.3 g of this product are suspended in 30 ml of water at 0°C.

The suspension is cooled by the exterior application of ice and nitrogen is bubbled through the mixture. By addition of a 1 N sodium hydroxide solution, until a pH-value of 7 is obtained, the precipitate dissolves. 90 ml of ethanol and 1.2 g of activated carbon are added and the reaction mixture is stirred at 0°C for ½ hour. The mixture is filtered and the filtrate is concentrated in vacuo (bath temperature not exceeding 20°C), during which 2 portions of 80 ml of dry ethanol are added to remove as much water as possible. When concentrated to about 15 ml the residue is, with stirring, poured into a mixture of 55 ml of 2,2-dimethoxypropane and 120 ml of acetone. The resulting precipitate is filtered off, washed with acetone and dried in vacuo, yielding 2.4 g of the disodium title compound.

Example 2.

Preparation of the disodium salt of D - 6 - [$\alpha${ - 3 - (hydroxy(ethoxy)phosphinyl)ureido}- p - hydroxybenzylcarboxamido] - penicillanic acid.

D - 6 - ($\alpha$ - amino) - p - hydroxybenzylcarboxamidopenicillanic acid (amoxicillin) (108.5 g, 0.2975 mole) is silylated with 162.5 ml (0.655 mole) of N,O-bistrimethylsilylacetamide in 750 ml of methylene chloride. After cooling the solution to about −70°C a solution of 50.5 g (0.2975 mole) of ethylchlorophosphorusisocyanatidate in 750 ml of ethylacetate is added dropwise. After completion of the addition the mixture is stirred for 1½ hours and 25 ml (1.39 mole) of water and 1 l of ethylacetate are added at a temperature of −70°C. The temperature is allowed to rise to −40°C in 1 hour and then to 0°C in 1½ hours. After separation of the layers the organic layer is washed twice with 200 ml of water. The 600 ml 1N sodium hydroxide are added slowly at a pH-value of 5 or slightly lower. After completion of the addition the pH-value of the waterlayer is

brought to 7 with 1N sodium hydroxide in 1 hour. The solution is treated with 10 g of activated carbon, and, after filtration, concentrated in vacuo during which several portions of dry ethanol are added in order to remove as much water as possible. The residue is dissolved in 1½ l of methanol and concentrated in the same manner to 750 ml after which ethanol is added slowly during the concentration, thus keeping the volume 750 ml, till almost all methanol is removed. The precipitate formed is filtered off, washed with ethanol and diethylether and dried in vacuo over phosphoruspentoxide, yielding 100 g (60%) of the disodium title compound.

Example 3.

Preparation of the disodium salt of D - 6 - {$\alpha$ - [3 - hydroxy(ethoxy)phosphinyl)ureido]-benzylcarboxamido} - penicillanic acid.

10.5 g (0.03 mole) of D - 6 - ($\alpha$ - amino)benzylcarboxamidopenicillanic acid (ampicillin) is silylated with 9.6 ml (0.04 mole) of N,O-bistrimethylsilylacetamide in 50 ml of dry methylene chloride. The solution is cooled to −65°C and a solution of 5 g (0.03 mole) of $(C_2H_5O)PO(Cl)NCO$ (ethylchlorophosphorus-isocyanatidate) in 55 ml dry methylene chloride is added dropwise. The mixture is stirred for 75 minutes and then a mixture of 1.26 ml (0.07 mole) of water, 2.4 ml (0.03 mole) of pyridine and 30 ml of acetone is added. After completion of the addition the temperature is raised rapidly to −35°C and then slowly to 0°C over a period of time of 2 hours. A solution of 4.9 g (0.03 mole) of sodium 2-ethylhexanoate in 30 ml of dry acetone is added to the reaction mixture at 0°C, and the mixture is stirred for 1½ hours, the temperature being allowed to rise to about ambient temperature. A precipitate is formed which is filtered off and suspended in 150 ml of dry acetone. The suspension is stirred for 45 minutes and the precipitate is filtered off. This procedure is repeated once and the precipitate obtained is suspended in 100 ml of water. The suspension is cooled with ice, nitrogen is bubbled through the mixture and the precipitate is dissolved by the addition of an amount of sodium bicarbonte just sufficient for the desired salt formation. The solution obtained is freeze-dried, resulting in a yield of 8.6 g (53%) of the title compound having the following physicochemical properties:—

IR (Kbr-disc, values in $cm^{-1}$): ± 3550, ± 2600, ± 3320 and ± 3250, 1780, 1740—1710, 1640—1670, ± 1530 (intense), 1210, 1040, 700.

PMR ($d_6$-DMSO, 60 Mc, $\delta$ - values in ppm, DSS as reference): 1.2 (t, J ≈ 7.0 cps, 3H), 1.44 and 1.58 (6H), 3.95 (multiplet) and 4.24 (s) together 3H, about 5.5 (multiplet, J 5,6 ≈ 4.0 cps) and about 5.65 (d) together 3H, about 7.4 (5H), 7.7 (d, J ≈8.5 cps), 7.9 (d, J ≈ 7.5 cps), 9.15 (d, J ≈ 7.5 cps).

Example 4.

Preparation of disodium salts of other hydroxyphosphinylureidobenzylpenicillins.

In a similar manner to that described in Example 1 or 3 the following compounds are prepared in yields varying from 35 to 65%:

Disodium salt of D - 6 - {$\alpha$ - [3 - (hydroxy(methoxy) - phosphinyl)ureido] - *p* - hydroxybenzylcarboxamido} penicillanic acid having the following physicochemical properties:— IR (KBr—disc, values in $cm^{-1}$): about 3280—3600 (broad and intensive), ± 2950 (sh), 1760, 1680, (sh), about 1645 to 1665, ± 1600, ± 1540, 1500, 1455, 1395, 1370 (sh), 1345 (sh), 1310—1330, 1215—1245, 1180 (sh), 1125, 1080 (intensive), 1045, 895, ± 770.

PMR (about 5:1 mixture of $d_6$-DMSO and $DCO_2D$, 60 Mc, DSS as reference, $\delta$-values in ppm): 1.47 and 1.59 (6H), 3.50 (d, J ≈ 11.6 cps, 3H), 4.27 (s, 1H), 5.44 (s) and about 5.35 to 5.6 (broadened AB-q) together 3H, 6.7 to 7.35 (q-like, 4H).

Disodium salt of D-6-{$\alpha$ - [3 - (hydroxy(benzyloxy) - phosphinyl)ureido] - *p* - hydroxybenzylcarboxamido}penicillanic acid having the following physicochemical properties:— IR (KBr-disc, values in $cm^{-1}$): about 3100—3600, shoulders at ± 3050, 2970 and 2935, 1765, 1690 (sh), 1640—1660, 1595—1615, ± 1550 (sh), 1515, 1455, 1400, 1380 (sh), 1320—1340, 1220—1260, 1180, 1135, 1090 (intensive), 1010—1035, 985, 900, 870, 845, 750, 710.

PMR (about 4:1 mixture of $d_6$-DMSO and $DCO_2D$, 60 Mc, DSS as reference, $\delta$-values in ppm): 1.48 and 1.60 (6H), 4.26 (s, 1H), 4.86 (d, J ≈ 7.0 cps, 2H), 5.45 (s) and 5.35 to 5.60 (AB-q, J ≈ 4.0 cps) together 3H); 6.65 to 7.3 (q-like, J ≈ 8.2 cps) and about 7.35 together 9H.

Thin layer chromatography Rf about 0.9 (UV positive) (silica, 95:5:5 mixture of methanol, acetic acid and water).

Disodium salt of D - 6 - {$\alpha$ - [3 - hydroxy-(phenoxy) - phosphinyl)ureido] - *p* - hydroxy-benzylcarboxamido]penicillanic acid having the following physicochemical properties:—

IR (KBr-disc, values in $cm^{-1}$): 3400, 1780, 1660, 1610, 1520, 1400, 1320, 1220, 1140, 1060, 780, 700.

PMR (mixture of $d_6$-DMSO and $DCO_2D$, $\delta$-values in ppm, TMS as reference): 1.45 (s, 3H), 1.58 (s, 3H), 4.25 (s, 1H), 5.3—5.6 (multiplet, 3H), 6.75 (d, 2H) and 7.1—7.4 (multiplet, 7H).

The physicochemical properties of the three above-mentioned compounds are identical to those of the compounds prepared in Examples 5, 6 and 10 respectively of German "Offenlegungsschrift" No. 25 46 910.

Example 5.

Preparation of the trisodium salt of D - 6 - {$\alpha$ - [3 - (dihydroxyphosphinyl)ureido] - p -

hydroxybenzylcarboxamido} - penicillanic acid.

D - 6 - ($\alpha$ - amino) - p - hydroxybenzylcarboxamidopenicillanic acid (amoxicillin) (2.17 g, 5.95 mmoles) is silylated with 3.24 ml (13.1 mmoles) of N,O-bistrimethylsilylacetamide in 15 ml of methylene chloride as a solvent. After cooling the solution to about −75°C a solution of 0.95 g (5.95 mmoles) of dichlorophosphorusisocyanatidate in 15 ml of ethyl acetate is added dropwise in one hour. After completion of the addition the mixture is stirred for 1½ hours and a mixture of 0.96 ml (11.9 mmoles) of pyridine and 15 ml of ethyl acetate is added, followed by the addition of 0.69 ml (38 mmoles) of distilled water at a temperature of −70°C. The temperature of the reaction mixture is allowed to rise to −40°C in one hour and from −40° to 0°C in 1½ hours. At that temperature the reaction mixture is washed 3 times with 25 ml portions of acidified water at a pH-value of 1.5. Then 25 ml of water are added and the pH-value is brought slowly to 7 with 1N sodium hydroxide. The layers formed are separated and the aqueous layer is freeze-dried, yielding 3.0 g of a powder of the title compound, having a purity of 80%.

**Claims**

1. Process for the preparation of compounds of the general formula:—

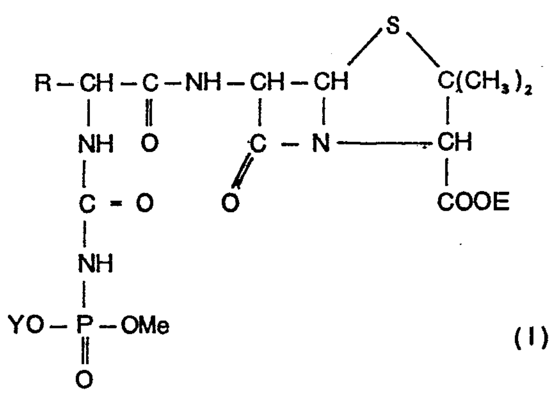

(I)

wherein R represents a phenyl group which may be substituted by one or two groups, which may be the same or different, selected from hydroxy, lower alkyl and lower alkoxy groups, Y represents a group Me or a lower alkyl, aryl(lower)alkyl or aryl group, in which the aryl group may be substituted by one or more lower alkyl groups, Me represents a metal cation and E represents a hydrogen atom or a metal cation, which may be the same or different from the metal cation Me, which comprises reacting a compound of the general formula:—

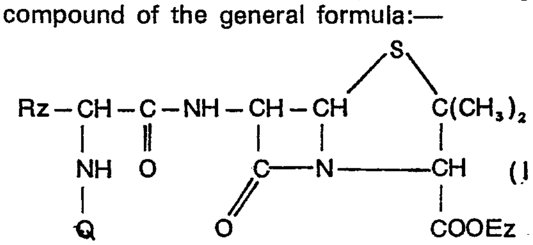

(II)

wherein Q represents a hydrogen atom or a silicon atom carrying substituents selected from lower alkyl, lower haloalkyl, aryl, aralkyl or lower alkoxyalkyl groups and halogen atoms, Rz has the same significance as R as hereinbefore defined, with the proviso that any hydroxy group(s) present on the phenyl group is (or are) replaced by a group —OQz, wherein Qz has the same significance as Q as hereinbefore defined, but excluding hydrogen, and Ez represents a carboxyl-protecting group, with a compound of the general formula:

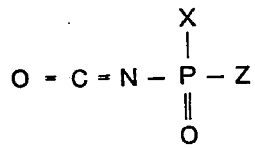

(III)

wherein Z represents a halogen atom, and X represents a group OY, wherein Y is as hereinbefore defined, or a group Z as hereinbefore defined, at a temperature below 0°C, under anhydrous conditions in an organic solvent medium characterized by carefully hydrolysing the product thus obtained either with an amount of water just sufficient to remove any protecting groups present in the intermediate product and to hydrolyse the group(s) Z to hydroxy group(s) and, still under anhydrous or substantially anhydrous conditions, converting hydroxy group(s) formed by hydrolysis of group(s) Z into group(s) OMe, wherein Me is as hereinbefore defined, by means of an organic acid salt having a salt-forming cation Me or, in which case the organic solvent used is one insoluble or substantially insoluble in water, with up to 100% excess of water, washing the mixture with water at a pH-value of 0 to 3, extracting the organic layer formed with water at a pH-value of 0 to 3, extracting the organic layer formed with water at a pH-value of 5 to 8 by means of adding a hydroxide or a salt having a salt-forming cation Me, thus converting hydroxy group(s) formed by hydrolysis of groups Z into groups OMe and Ez into Me, wherein Me is as hereinbefore defined.

2. Process according to claim 1, characterized in that the temperature for reacting compound (II) with compound (III) is kept at −90° to −40°C.

3. Process according to claim 1, characterized in that Q represents a trimethylsilyl group.

4. Process according to claim 1, characterized in that Z represents a chlorine atom.

5. Process according to claim 1, characterized in that E and Me represent an alkali metal or alkaline earth metal cation.

6. Process according to claim 5, characterized in that the cation is a sodium, potassium or calcium cation.

7. Process according to claim 1, characterized in that the organic acid salt having a salt-forming cation Me is an alkanoic acid salt,

the alkanoyl group of which containing 1 to 20 carbon atoms.

8. Process according to claim 7, characterized in that the alkanoyl group contains 5 to 10 carbon atoms.

9. Process according to claim 7, characterized in that the organic acid salt is a salt of acetic, propionic, a butyric, a pentanoic, a hexanoic, a heptanoic, an octanoic or stearic acid.

10. Process according to claim 9, characterized in that the organic acid salt is a salt of 2-ethylhexanoic acid.

11. Process according to claim 1, characterized in that the salt formation is carried out in an organic solvent having moderately polar properties.

**Revendications**

1. Procédé de préparation de composés de formule générale:

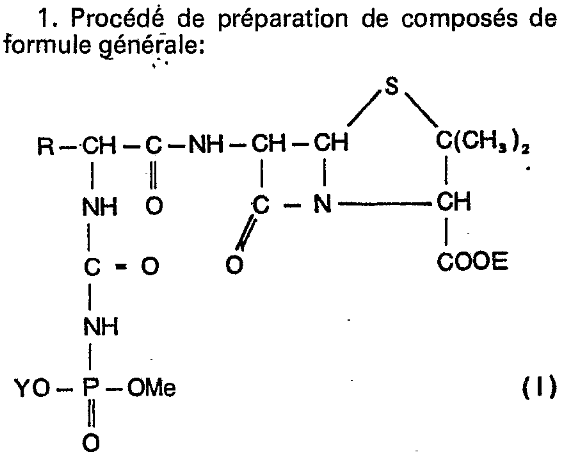

(I)

où R représente un radical phényle qui peut porter comme substituant un ou deux radicaux, identique ou différents, choisis parmi les radicaux hydroxyle, alkyle inférieurs et alkoxy inférieurs, Y représente un radical Me ou un radical alkyle inférieur, arylalkyle à radical alkyle inférieur ou aryle, le radical aryle pouvant porter comme substituant un ou plusieurs radicaux alkyle inférieurs, Me représente un cation métallique et E représente un atome d'hydrogène ou un cation métallique, lequel peut être identique ou non au cation métallique Me, suivant lequel on fait réagir un composé de formule générale:

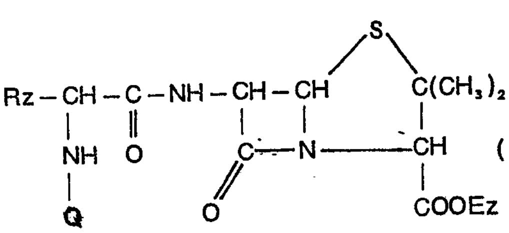

(II)

où Q représente un atome d'hydrogène ou un atome de silicium portant des substituants choisis parmi les radicaux alkyle inférieurs, halogénoalkyle inférieurs, aryle, aralkyle et alkoxyalkyle inférieurs et les atomes d'halogène, Rz a la même signification que R ci-dessus, avec la restriction que le ou le radicaux hydroxyle éventuels portés par le radical phényle sont remplacés par un radical de formule —OQz où Qz a la même signification que Q ci-dessus, mais à l'exclusion de l'atome d'hydrogène et Ez représente un radical protecteur du radical carboxyle, avec un composé de formule générale:

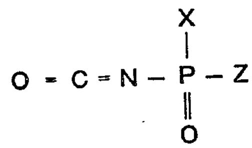

(III)

où Z représente un atome d'halogène et X représente un radical OY, où Y est tel que défini ci-dessus, ou un radical Z tel que défini ci-dessus à une température inférieure à 0°C en milieu anhydre dans un solvent organique, caractérisé en ce qu'on hydrolyse prudemmant le produit résultant soit avec une quantité d'eau juste suffisante pour éliminer les radicaux protecteurs éventuels du produit intermédiaire et hydrolyser le ou les radicaux Z en un ou des radicaux hydroxyle et, tandis que le milieu est encore anhydre ou sensiblement anhydre, on convertit le ou les radicaux hydroxyle issus de l'hydrolyse du ou des radicaux Z en un ou des radicaux de formule OMe, où Me est tel que défini ci-dessus, au moyen d'un sel d'un acide organique apportant un cation Me salifiant, soit, auquel cas où le solvant organique utilisé est insoluble ou sensiblement insoluble dans l'eau, avec une quantité d'eau en un excès s'élevant jusqu' à 100%, on lave le mélange avec de l'eau à un pH de 0 à 3, on extrait la couche organique formée avec de l'eau à un pH de 5 à 8 par une addition d'un hydroxyde ou d'un sel apportant un cation Me salifiant, convertissant ainsi le ou les radicaux hydroxyle issus de l'hydrolyse des radicaux Z en radicaux OMe et les radicaux Ez en radicaux Me, Me ayant la signification qui lui a été donnée ci-dessus.

2. Procédé suivant la revendication 1, caractérisé en ce que la température pour la réaction du composé de formule (II) avec le composé de formule (III) est maintenue à une valeur de —90 à —40°C.

3. Procédé suivant la revendication 1, caractérisé en ce que Q représente un radical triméthylsilyle.

4. Procédé suivant la revendication 1, caractérisé en ce que Z représente un atome de chlore.

5. Procédé suivant la revendication 1, caractérisé en ce que E et Me représentent un cation de métal alcalin ou alcalino-terreux.

6. Procédé suivant la revendication 5, caractérisé en ce que le cation est un cation sodium, potassium ou calcium.

7. Procédé suivant la revendication 1, caractérisé en ce que le sel d'acide organique

apportant un cation Me salifiant est un sel d'acide alkanoïque dont le radical alkanoyle compte 1 à 20 atomes de carbone.

8. Procédé suivant la revendication 7, caractérisé en ce que le radical alkanoyle compte 5 à 10 atomes de carbone.

9. Procédé suivant la revendication 7, caractérisé en ce que le sel d'acide organique est un sel d'acide acétique, d'acide propionique, d'un acid butyrique, d'un acid pentanoïque, d'un acide hexanoïque, d'un acid heptanoïque, d'un acide octanoïque ou d'acide stérique.

10. Procédé suivant la revendication 9, caractérisé en ce que le sel d'acide organique est un sel d'acide 2-ethylhexanoïque.

11. Procédé suivant la revendication 1, caractérisé en ce que la formation du sel est exécutée dans un solvant organique ayant des propriétés modérément polaires.

**Ansprüche**

1. Verfahren zur Herstellung von Verbindungen der allgemeinen Formel:

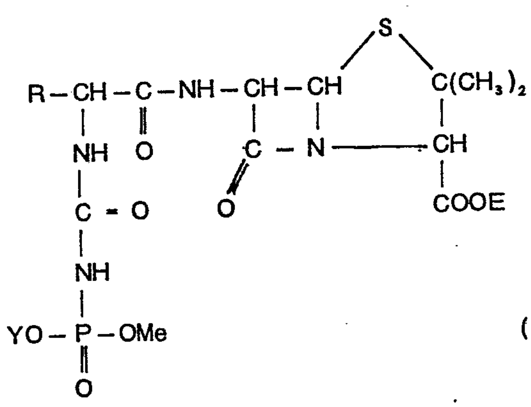

(I)

inder R eine gegebenenfalls durch eine oder zwei Reste substituierte Phenylgruppe bedeutet, wobei diese Reste gleich oder verschieden sind und eine Hydroxylgruppe, einen Niederalkyl- oder Niederalkoxyrest bedeuten, Y den Rest Me, einen Niederalkyl-, Arylniederalkyl- oder Arylrest bedeutet, wobei der Arylrest gegebenenfalls durch eine oder mehrere Niederalkylreste substituiert ist und Me ein Metallkation bedeutet, sowie E ein Wasserstoffatom oder ein Metallkation darstellt, das gleich dem Metallkation Me ist oder ein davon verschiedenes Metallkation bedeutet, worin man eine Verbindung der allgemeinen Formel:

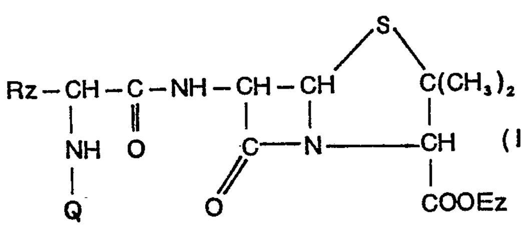

(II)

in der Q ein Wasserstoffatom oder ein Siliciumatom bedeutet, das mindestens einen Substituenten aus der Reihe Niederalkyl-, Niederhalogenalkyl-, Aryl-, Aralkyl- oder Niederalkoxyalkylrest oder Halogenatom trägt, Rz die für R angegebene Bedeutung hat, mit der Massgabe, dass jede der an die Phenylgruppe gebundenen Hydroxylgruppen durch die Gruppe- OQz ersetzt ist, wobei Qz die für Q angegebene Bedeutung, ausgenommen Wasserstoff, hat, und Ez eine Carboxylschutzgruppe darstellt, mit einer Verbindung der allgemeinen Formel:

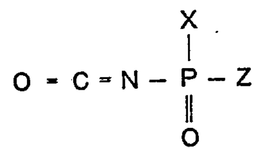

(III)

in der Z ein Halogenatom und X den Rest OY oder den vorgenannten Rest Z darstellen, wobei Y die vorstehende Bedeutung hat, bei einer Temperatur unterhalb 0°C unter wasserfreien Bedingungen in einem organischen Lösungsmittel umsetzt, dadurch gekennzeichnet, dass man das erhaltene Produkt vorsichtig dadurch hydrolysiert, dass man es mit einer zur Abspaltung der in dem Zwischenprodukt vorliegenden Schutzgruppen gerade ausreichenden Menge Wasser umsetzt, um die Reste Z in Hydroxylgruppen zu überführen sowie unter immer noch wasserfreien oder im Wesentlichen wasserfreien Bedingungen die durch Hydrolyse aus den Resten Z gebildeten Hydroxylgruppen in Reste OMe umwandelt, wobei Me die vorstehende Bedeutung hat und diese Umwandlung mit einem Salz einer organischen Säure erfolgt, das ein salzbildendes Kation Me aufweist, oder das erhaltene Produkt vorsichtig dadurch hydrolysiert, dass man es in einem in Wasser unlöslichen oder im wesentlichen unlöslichen organischen Lösungsmittel mit bis zu 100%igem Überschuss Wasser umsetzt, das Gemisch bei einem pH-Wert von 0 bis 3 mit Wasser wäscht, die gebildete organische Schicht bei einem pH-Wert von 5 bis 8 unter Zugabe eines Hydroxids oder eines ein Salz bildendes Kation Me enthaltenden Salzes mit Wasser extrahiert, wobei die durch Hydrolyse der Reste Z gebildeten Hydroxylgruppen in Reste OMe und die Reste Ez in Reste Me überführt werden, wobei Me die vorstehende Bedeutung hat.

2. Verfahren nach Anspruch 1, dadurch gekennzeichnet, dass man die Verbindung der allgemeinen Formel (II) mit der Verbindung der allgemeinen Formel (III) bei Temperaturen von —90 bis —40°C umsetzt.

3. Verfahren nach Anspruch 1, dadurch gekennzeichnet, dass man solche Verbindungen einsetzt, in denen Q eine Trimethylsilylgruppe bedeutet.

4. Verfahren nach Anspruch 1, dadurch gekennzeichnet, dass man solche Verbindungen einsetzt, in denen Z ein Chloratom bedeutet.

5. Verfahren nach Anspruch 1, dadurch

gekennzeichnet, dass man solche Verbindungen einsetzt, in denen E und Me jeweils ein Alkalimetalloder Erdalkalimetallkation bedeuten.

6. Verfahren nach Anspruch 5, dadurch gekennzeichnet, dass man solche Verbindungen einsetzt, in denen E und Me jeweils ein Natrium-, Kalium- oder Calciumkation bedeuten.

7. Verfahren nach Anspruch 1, dadurch gekennzeichnet, dass man als Salz einer organischen Säure, das ein salzbildendes Kation Me enthält, ein Salz einer Alkancarbonsäure einsetzt, deren Alkanoylgruppe 1 bis 20 Kohlenstoffatome enthält.

8. Verfahren nach Anspruch 7, dadurch gekennzeichnet, dass man solche Salze einer Alkancarbonsäure einsetzt, deren Alkanoylgruppe 5 bis 10 Kohlenstoffatome enthält.

9. Verfahren nach Anspruch 7, dadurch gekennzeichnet, dass man als Salz einer organischen Säure ein Salz der Essig- oder Propionsäure oder einer Butancarbon-, Pentancarbon-, Hexancarbon-, Heptancarbon-, Octancarbon- oder Stearinsäure einsetzt.

10. Verfahren nach Anspruch 9, dadurch gekennzeichnet, dass man als Salz einer organischen Säure ein Salz der 2-Äthylhexancarbonsäure einsetzt.

11. Verfahren nach Anspruch 1, dadurch gekennzeichnet, dass man die Salzbildung in einem organischen Lösungsmittel mit mässig polaren Eigenschaften durchführt.